# EUROPÄISCHE PATENTANMELDUNG

(11) **EP 1 424 323 A1**
(43) Veröffentlichungstag der Anmeldung: **02.06.2004**
(21) Anmeldenummer: 03026516.9
(22) Anmeldetag: 18.11.2003
(51) Int. Cl.: C07C 253/30, C07C 255/54

(54) **Verfahren zur Herstellung von 3-Alkoxyphthalsäuredinitrilen**

(30) Priorität: 29.11.2002 DE 10256105
(71) Anmelder: Bayer Chemicals AG, 51368 Leverkusen (DE)
(72) Erfinder: Berneth, Horst, Dr., 51373 Leverkusen (DE); Bruder, Friedrich-Karl, Dr., 47802 Krefeld (DE); Hagen, Rainer, Dr., 51373 Leverkusen (DE); Hassenrück, Karin, Dr., 40468 Düsseldorf (DE); Kostromine, Serguei, Dr., 53913 Swisttal (DE); Krüger, Christa Maria, Dr., 48149 Münster (DE); Meyer-Friedrichsen, Timo, Dr., 47800 Krefeld (DE); Stawitz, Josef-Walter, Dr., 51519 Odenthal (DE); Oser, Rafael, Dr., 47800 Krefeld (DE); Falkner, Oliver, 50769 Köln (DE)

(57) **Zusammenfassung**

Verfahren zur Herstellung von 3-Alkoxyphthalsäuredinitrilen der Formel I 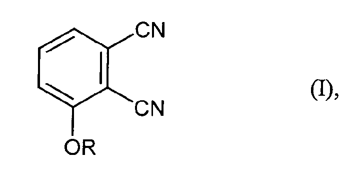 worin
- R: für gegebenenfalls substituiertes Alkyl oder Cycloalkyl steht,
durch Umsetzung von 3-Nitrophthalsäuredinitril mit einem Alkohol ROH, in einem von den Reaktanden verschiedenen Lösungsmittel in Gegenwart einer Base, dadurch gekennzeichnet, dass entweder
a) 3-Nitrophthalsäuredinitril und die Base in einem Lösungsmittel vorgelegt werden und der Alkohol ROH der Vorlage zugegeben wird, oder
b) der Alkohol ROH und die Base in einem Lösungsmittel vorgelegt werden und 3-Nitrophthalsäuredinitril der Vorlage zugegeben wird.

## Beschreibung

Die Erfindung betrifft ein Verfahren zur Herstellung von 3-Alkoxyphthalsäuredinitrilen.

3-Alkoxyphthalsäuredinitrile sind wichtige Vorprodukte zur Herstellung von lichtabsorbierenden Verbindungen, die in der Informationsschicht von optischen. Datenträgem verwendet werden.

Die Herstellung von 3-(2,4-Dimethyl-3-pentoxy)phthalodinitril ist beispielsweise bereits von I. Renge et al. J. Phys. Chem. A 1997, 101, 6202-6213 beschrieben worden.

Dabei wird 3-Nitrophthalsäuredinitril zusammen mit 2,4-Dimethyl-3-pentanol in DMF zunächst vorgelegt und dazu portionsweise NaH zugegeben. Die Ausbeute ist mit 78,5 % allerdings nicht zufriedenstellend.

Aufgabe der vorliegenden Erfindung war es, ein verbessertes Verfahren mit einer höheren Ausbeute bereitzustellen.

Die Erfindung betrifft daher ein Verfahren zur Herstellung von 3-Alkoxyphthalsäuredinitrilen der Formel I 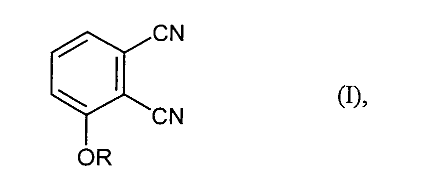 worin
- R: für gegebenenfalls substituiertes Alkyl oder Cycloalkyl steht,
durch Umsetzung von 3-Nitrophthalsäuredinitril mit einem Alkohol ROH, in einem von den Reaktanden verschiedenen Lösungsmittel in Gegenwart einer Base, dadurch gekennzeichnet, dass entweder
a) 3-Nitrophthalsäuredinitril und die Base in einem Lösungsmittel vorgelegt werden und der Alkohol ROH der Vorlage zugegeben wird, oder
b) der Alkohol ROH und die Base in einem Lösungsmittel vorgelegt werden und 3-Nitrophthalsäuredinitril der Vorlage zugegeben wird.

In einer bevorzugten Ausführungsform des erfindungsgemäßen Verfahrens kann der Alkyl- oder Cycloalkyl-Rest weitere Reste wie Halogen, Hydroxy, Hydroxyalkyl, Amino, Alkylamino, Dialkylamino, Nitro, Cyano, CO-NH₂, Alkoxy, Alkoxycarbonyl, Morpholino, Piperidino, Pyrrolidino, Pyrrolidono, Trialkylsilyl, Trialkylsiloxy oder substituiertes oder unsubstituiertes Phenyl tragen. Der Alkylrest kann zudem mit einem Cycloalkylrest substituiert sein und der Cycloalkylrest mit einem Alkylrest. Der Alkyl- oder Cycloalkylrest kann gesättigt, ungesättigt, geradkettig oder verzweigt sein, der Alkyl- oder Cycloalkylrest kann teil- oder perhalogeniert sein oder er kann ethoxyliert, propoxyliert oder silyliert sein.

Substituenten mit der Bezeichnung "Alkyl" bedeuten vorzugsweise C₁-C₁₆-Alkyl, insbesondere C₁-C₁₂-Alkyl, besonders bevorzugt C₁-C₈-Alkyl bedeuten, die gegebenenfalls durch Halogen, wie Chlor, Brom, Fluor, Hydroxy, Cyano und/oder C₁-C₆-Alkoxy substituiert sind.

Substituenten mit der Bezeichnung "Cycloalkyl" bedeuten vorzugsweise C₃-C₁₂-Cycloalkyl, insbesondere C₅-C₈-Cycloalkyl bedeuten, die gegebenenfalls durch Halogen, wie Chlor, Brom, Fluor, Hydroxy, Cyano und/oder C₁-C₆-Alkoxy substituiert sind.

In einer besonders bevorzugten Ausführungsform steht der Rest
- R: für Methyl, Ethyl, Propyl, Isopropyl, Butyl, Isobutyl, tert.-Butyl, Pentyl, 3-(2,4-Dimethyl)pentyl, tert.-Amyl, Hexyl, Heptyl, Octyl, Nonyl, Decyl, Undecyl, Dodecyl, Ethylhexyl, Hydroxyethyl, Methoxyethyl, Ethoxyethyl, 3-(2-Ethylhexyloxy)propyl, Methoxyethoxypropyl, Methoxyethoxyethyl, 3-Dimethylaminopropyl, 3-Diethylaminopropyl, Cyclopentyl, Cyclohexyl, Phenylcyclohexyl oder Cyclooctyl, insbesondere für 3-(2,4-Dimethyl)pentyl.

Als geeignete Base kommen beispielsweise Alkalicarbonate, Erdalkalicarbonate, Alkalihydroxide, Erdalkalihydroxyde, Diazabicycloundecan (DBU), Alkalihydride oder Erdalkalihydride, insbesondere Lithiumcarbonat, Kaliumcarbonat, Natriumcarbonat, Lithiumhydroxid, Kaliumhydroxid, Natriumhydroxid, DBU, Lithiumhydrid, Kaliumhydrid oder Natriumhydrid in Frage.

Als geeignete Lösungsmittel kommen beispielsweise DMF, NMP, DMSO oder Mischungen davon in Frage.

Die Reaktion erfolgt vorzugsweise bei Temperaturen von -10°C bis 60°C, insbesondere von -5°C bis 50°C.

Bevorzugt ist das erfindungsgemäße Verfahren, bei dem der Alkohol ROH und die Base im Lösungsmittel vorgelegt werden, und 3-Nitrophthalsäuredinitril, gegebenenfalls gelöst im Lösungsmittel, zudosiert wird. Bevorzugte Temperaturen sind für die Umsetzung von -10°C bis 20°C, insbesondere von -5°C bis 15°C, ganz besonders bevorzugt -5°C bis +5°C.

Bevorzugt wird die Umsetzung in Gegenwart einer Inertgasatmosphäre wie beispielsweise N₂ oder Argon durchgeführt.

Die Zugabe des Alkohols ROH zur Vorlage erfolgt vorzugsweise in ein bis 5 vorzugsweise in ein bis 3 Portionen.

Das molare Verhältnis vin Alkohol ROH zur Nitroverbindung ist unkritisch. Das Alkohol kann dabei im Überschuss eingesetzt werden. Bevorzugt wird der Alkohol zur Nitroverbindung im molaren Verhältnis von 0.9 bis 1.1, besonders bevorzugt equimolar eingesetzt.

Die Ausbeute des erfindungsgemäßen Verfahrens sind deutlich höher als die des Standes der Technik.

### Beispiele

Die folgenden präparativen Beispiele zeigen die Herstellung der erfindungsgemäß zu verwendenden Farbstoffe.

### Beispiel 1

51 NMP werden in dem trockenen Reaktionsgefäß vorgelegt und mit N₂ überschleiert. Unter ständigem Stickstoffstrom wird auf 4°C abkühlt und mit 660g NaH (60 %ig in Mineralöl) versetzt. Dann werden 2025g 2-Ethylhexanol rasch zugegeben und auf 0°C gekühlt Bei dieser Temperatur werden 2622g 3-Nitrophthalodinitril in 101 NMP zudosiert. Der Ansatz wird auf Eis ausgetragen und abgesaugt. Das Produkt wird zuerst mit einem Gemisch aus MeOH und Wasser (2:1) in Portionen und dann mit Wasser gewaschen und trocken gesaugt. Nach dem Trocknen im Vakuum bei 30°C erhält man 3517g ( =̂ 91% der Theorie) Produkt der Formel:

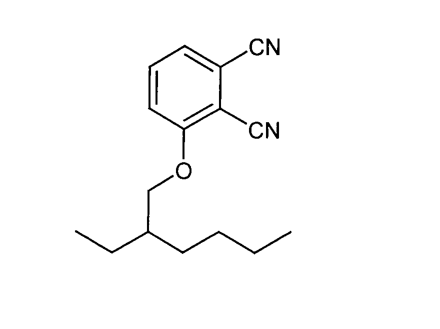

### Beispiel 2

2623.5 g 3-Nitrophthalodinitril und 15 1 NMP werden in dem trockenen Reaktionsgefäß vorgelegt und mit N₂ überschleiert. Unter ständigem Stickstoffstrom wird auf 4°C abkühlt und mit 660g NaH (60 %ig in Mineralöl) versetzt. Dann werden 1 kg 2,4-Dimethyl-3-pentanol rasch zugegeben. Innerhalb von 1h steigt die Temperatur bis auf 23°C an. Danach werden weitere 2024g 2,4-Dimethyl -3-pentanol zugegeben.

Die Temperatur steigt dabei auf 40°C an und wird 20 min gehalten. Der Ansatz wird auf 10°C abgekühlt, auf Eis ausgetragen und abgesaugt. Das Produkt wird zuerst mit Gemisch aus MeOH und Wasser (2:1) in Portionen und dann mit Wasser gewaschen und trocken gesaugt. Nach dem Trocknen im Vakuum bei 30°C erhält man 3080g (=̂ 85 der Theorie) Produkt der Formel:

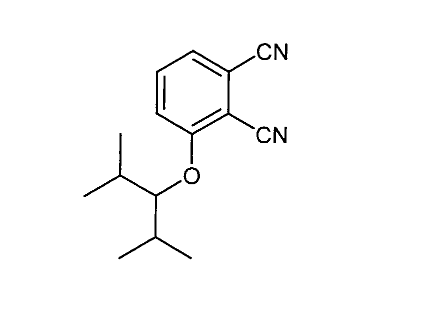

### Beispiel 3

7.41 DMF werden in dem trockenen Reaktionsgefäß vorgelegt und mit N₂ überschleiert. Dann werden 2047g 2,4-Dimethyl-3-pentanol rasch zugegeben und auf 0°C gekühlt. Unter ständigem Stickstoffstrom wird 669g NaH (60 %ig in Mineralöl) zugegeben. Bei dieser Temperatur werden 2623g 3-Nitrophthalodinitril in 2.31 DMF zudosiert. Der Ansatz wird auf Eis ausgetragen und abgesaugt. Das Produkt wird zuerst mit einem Gemisch aus MeOH und Wasser (2:1) in Portionen gewaschen und dann mit Petrolether gewaschen und trocken gesaugt. Nach dem Trocknen im Vakuum bei 30°C erhält man 3275g (=̂ 89% der Thoerie) Produkt der Formel:

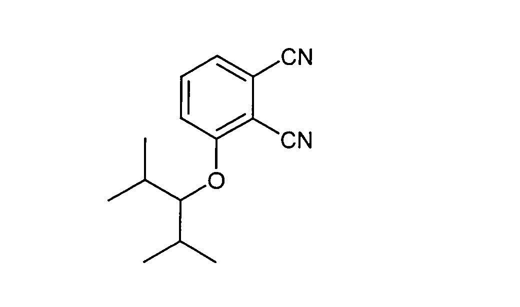

## Patentansprüche

1. Verfahren zur Herstellung von 3-Alkoxyphthalsäuredinitrilen der Formel I 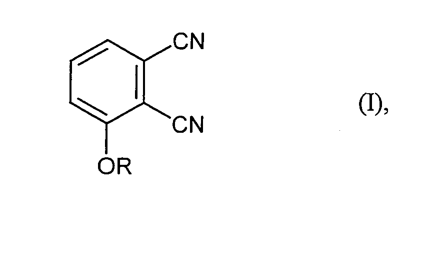 worin
R für gegebenenfalls substituiertes Alkyl oder Cycloalkyl steht,
durch Umsetzung von 3-Nitrophthalsäuredinitril mit einem Alkohol ROH, in einem von den Reaktanden verschiedenen Lösungsmittel in Gegenwart einer Base, **dadurch gekennzeichnet, dass** entweder
a) 3-Nitrophthalsäuredinitril und die Base in einem Lösungsmittel vorgelegt werden und der Alkohol ROH der Vorlage zugegeben wird, oder
b) der Alkohol ROH und die Base in einem Lösungsmittel vorgelegt werden und 3-Nitrophthalsäuredinitril der Vorlage zugegeben wird.

2. Verfahren gemäß Anspruch 1, wobei als Base Lithiumcarbonat, Kaliumcarbonat, Natriumcarbonat, Lithiumhydroxid, Kaliumhydroxid, Natriumhydroxid, DBU, Lithiumhydrid, Kaliumhydrid oder Natriumhydrid, insbesondere Natriumhydrid eingesetzt wird.

3. Verfahren gemäß Anspruch 1, **dadurch gekennzeichnet, dass**
R für Methyl, Ethyl, Propyl, Isopropyl, Butyl, Isobutyl, tert. Butyl, Pentyl, 3-(2,4-Dimethyl)pentyl, tert.-Amyl, Hexyl, Heptyl, Octyl, Nonyl, Decyl, Undecyl, Dodecyl, Ethylhexyl, Hydroxyethyl, Methoxyethyl, Etoxyethyl, 3-(2-Ethylhexyloxy)propyl, Methoxy-ethoxypropyl, Methoxyethoxyethyl, 3-Dimethylaminopropyl, 3-Di-ethylaminopropyl, Cyclopentyl, Cyclohexyl, Phenylcyclohexyl oder Cyclooctyl, insbesondere für 3-(2,4-Dimethyl)pentyl
steht.

4. Verfahren gemäß Anspruch 1, **dadurch gekennzeichnet, dass** man als Lösungsmittel DMF, NMP, DMSO oder Mischungen davon eingesetzt.

5. Verfahren gemäß Anspruch 1, **dadurch gekennzeichnet, dass** die Umsetzung bei einer Temperatur von -10 bis 60°C erfolgt.

6. Verfahren gemäß Anspruch 1, **dadurch gekennzeichnet, dass** der Alkohol ROH und die Base im Lösungsmittel vorgelegt werden und 3-Nitrophthalsäuredinitril, gegebenenfalls gelöst im Lösungsmittel der Vorlage zugegeben wird.

7. Verfahren gemäß Anspruch 1, **dadurch gekennzeichnet, dass** 3-Nitrophthalsäuredinitril und die Base in einem Lösungsmittel vorgelegt werden und der Alkohol ROH der Vorlage zugegeben wird.
